# EUROPEAN PATENT SPECIFICATION

(11) **EP 1 917 014 B1**
(45) Date of publication and mention of the grant of the patent: **07.10.2009**
(21) Application number: 06801521.3
(22) Date of filing: 15.08.2006
(51) Int. Cl.: A61K 31/428, A61P 25/28

(54) **NEURORESTORATION WITH R(+) PRAMIPEXOLE**
NEURORESTAURATION MIT R(+) PRAMIPEXOL
NEURORESTAURATION AVEC DU PRAMIPEXOLE R(+)

(30) Priority: 15.08.2005 US 708213 P
(43) Date of publication of application: 07.05.2008
(73) Proprietor: UNIVERSITY OF VIRGINIA PATENT FOUNDATION, Charlottesville, VA 22902 (US)
(72) Inventor: BENNETT, James, P., Jr., Crozet, VA 22932 (US)
(74) Representative: HOFFMANN EITLE
(86) International application number: PCT/US2006/031831
(87) International publication number: WO 2007/022182

(56) References cited:
- WO-A-96/18395
- WO-A-20/05011687
- WO-A2-03/049705
- ABRAMOVA N A ET AL: "Inhibition by R(+) or S(-) pramipexole of caspase activation and cell death induced by methylpyridinium ion or beta amyloid peptide in SH-SY5Y neuroblastoma" JOURNAL OF NEUROSCIENCE RESEARCH 15 FEB 2002 UNITED STATES, vol. 67, no. 4, 15 February 2002 (2002-02-15), pages 494-500, XP009075274 ISSN: 0360-4012
- PATTEE G L ET AL: "Reduction of oxidative stress in amyotrophic lateral sclerosis following pramipexole treatment" AMYOTROPHIC LATERAL SCLEROSIS AND OTHER MOTOR NEURON DISORDERS 2003 UNITED KINGDOM, vol. 4, no. 2, 2003, pages 90-95, XP009075253 ISSN: 1466-0822

## Description

### Field of the Invention

The present invention relates to the use of pramipexole (2-amino-4,5,6,7-tetrahydro-6-propylaminobenzathiazole), and analogs and derivatives thereof, to restore neuronal, muscular (cardiac and striated) and/or retinal tissue function. More particularly, the invention is directed to the use of the substantially pure stereoisomer R-(+)-2-amino-4,5,6,7-tetrahydro-6-propylaminobenzathiazole, and pharmacologically acceptable salts thereof, to restore neuronal, muscular (cardiac and striated) and/or retinal tissue function, and formulations thereof.

### Background of the Invention

Many degenerative brain diseases in children and adults develop from the premature death of vulnerable neurons, giving rise to varying clinical symptoms and phenotypes. While substantial efforts have been devoted to developing therapies to retard these neurodegenerative processes, few therapies have been developed which provide neurorestoration and thus an improvement in clinical symptoms as opposed to simply slowing the decline of function.

Neurodegenerative diseases (NDD), such as Alzheimer's disease (AD) and Parkinson's disease (PD), arise from the accelerated loss of certain populations of neurons in the brain. PD and AD usually appear sporadically without any obvious Mendelian inheritance patterns, but may show maternal biases. Although rare or uncommon inherited forms of adult NDD exist, the relevance of pathogenesis in these autosomal genetic variants, versus the much more commonly occurring sporadic forms, has been the subject of intense debate.

Accumulating evidence suggests that a primary etiologic component of sporadic adult NDD is in fact mitochondrial dysfunction and the resulting increased cellular oxidative stress. PD and AD brains and non-CNS tissues show reductions in mitochondrial electron transport chain (ETC) activity. When selectively amplified in cytoplasmic hybrid ("cybrid") cell models, mitochondrial genes from PD (Swerdlow, et al, Exp Neurol., 153:135-42 (1998)) and AD (Swerdlow, et al, Exp Neurol., 153:135-42 (1997)) subjects recapitulate the ETC deficits, produce increased oxidative stress, and exhibit a variety of other significant mitochondrial and cellular dysfunctions. These studies suggest that compounds that relieve oxidative stress by scavenging oxygen free radicals may have potential as neuroprotective agents for these diseases (Beal, Exp Neurol., 153:135-42 (2000)).

Oxidative stress has also been associated with the fatal neurodegenerative disorder amyotrophic lateral sclerosis (ALS). ALS, also known as Lou Gehrig's disease, is a progressive, fatal neurodegenerative disorder involving the motor neurons of the cortex, brain stem, and spinal cord. It is a degenerative disease of upper and lower motor neurons that results in progressive weakness of voluntary muscles, and eventually death. The onset of disease is usually in the fourth or fifth decade of life, and affected individuals succumb within 2 to 5 years of disease onset. ALS occurs in both sporadic and familial forms.

About 10% of all ALS patients are familial cases, of which 20% have mutations in the superoxide dismutase 1 (SOD 1) gene (formerly known as Cu,Zn-SOD), suggesting that an abnormally functioning Cu,Zn-SOD enzyme may play a pivotal role in the pathogenesis and progression of familial amyotrophic lateral sclerosis (FALS). It is believed that the increased generation of oxygen free radicals, especially hydroxyl radicals, by mutant SOD1, initiates the sequence of events leading to motor neuron death in FALS. This hypothesis is supported by recent reports that transfection of neuronal precursor cells with mutant SOD1 results in increased production of hydroxyl radicals and enhanced rate of cell death by apoptosis. It has also been suggested that oxidative stress is responsible for motor neuron death in sporadic forms of ALS as well.

Recent research has revealed that a likely inciting event in the premature neuronal death that is associated with ALS is the presence of mutated mitochondrial genes (mitochondrial DNA, mtDNA). These mtDNA mutations lead to abnormalities in the energy production pathways in mitochondria, resulting in excessive generation of damaging oxygen derivatives known as "reactive oxygen species" (ROS), including species called "oxygen free radicals." When ROS production exceeds the capacity of cellular mechanisms to remove/inactivate ROS, the condition known as "oxidative stress" results.

No therapeutic agent presently exists that is capable of widespread cellular restoration in the conditions described above. There exists a need for compositions and methods useful for restoring neurological and motor function in subjects with neurodegenerative diseases such as ALS.

Therefore, it is an object of the invention to provide compositions useful for restoring neurological and motor function in patients suffering from neurodegenerative diseases, and methods of using thereof.

### Summary of the Invention

Formulations and methods of use thereof for restoring neuronal, muscular (cardiac and striated) and/or retinal tissue function in children and adults afflicted with chronic neurodegenerative diseases, such as neurodegenerative movement disorders and ataxias, seizure disorders, motor neuron diseases, and inflammatory demyelinating disorders, are described herein. Examples of disorders include Alzheimer's disease (AD), Parkinson's disease (PD), and amyotrophic lateral sclerosis (ALS).

The method involves administering a pharmaceutical composition containing an effective amount of a tetrahydrobenzathiazole having the formula shown below: 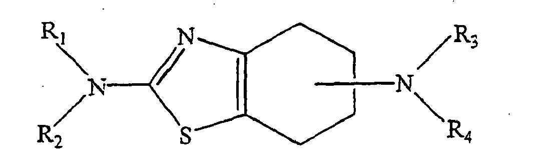 wherein R₁, R₂, R₃, and R₄, are independently selected from the group consisting of H, C₁-C₃ alkyl and C₁-C₃ alkenyl and the NR₃R₄ group is in the 6-position. In one embodiment, the tetrahydrobenzathiazole is the R(+) enantiomer of pramipexole in combination with the S(-) enantiomer of pramipexole. In another embodiment, the tetrahydrobenzathiazole is a substantially pure formulation of R(+) pramipexole, or biologically active analogs, derivatives, and homologs thereof, or pharmaceutically acceptable salts thereof. In yet another embodiment, the tetrahydrobenzathiazole is the R(+) enantiomer of pramipexole, or biologically active analogs, derivatives, and homologs thereof, or pharmaceutically acceptable salts thereof, substantially free of the S(-) enantiomer. The structural formulas of the R(+) and S(-) enantiomers of pramipexole are shown below.

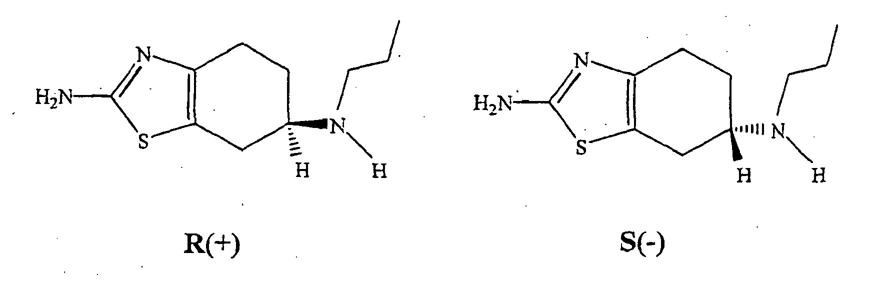

The compositions can be formulated for immediate and/or modified release. Modified release includes extended release, delayed release, and/or pulsatile release. The compositions can be administered by a variety of routes, including topical, transdermal, enteral, and parenteral (i.e. intravenous, subcutaneous or intramuscular) administration. Suitable oral dosage forms include gelatin and non-gelatin capsules, tablets, caplets, powders, lozenges, cachets, troches, syrups, solutions, suspensions, and emulsions. The compositions can also be implanted for immediate and/or modified release.

R(+) pramipexole is generally administered in doses ranging from 0.1-300 mg/kg/daily, preferably 0.5-50 mg/kg/daily, and most preferably 1-10 mg/kg/daily for oral administration. Daily total doses administered orally are typically between 10 mg and 500 mg. Alternatively, R(+) pramipexole can be administered parenterally to humans with acute brain injury in single doses between 10 mg and 100 mg, and/or by continuous intravenous infusions between 10 mg/day and 500 mg/day.

### Detailed Description of the Invention

### I. Definitions

"Neurorestorative", as used herein, refers to improvements in neural deficits, retinal function, and/or motor function.

As used herein, an "analog" of a chemical compound is a compound that, by way of example, resembles another in structure but is not necessarily an isomer (e.g., 5-fluorouracil is an analog of thymine).

As used herein, a "derivative" of a compound refers to a chemical compound that may be produced from another compound of similar structure in one or more steps. Derivatives generally involve the addition and/or modification of one or more functional groups on the parent compound.

As used herein, a "package insert" refers to a publication, a recording, a diagram, or any other medium of expression which can be used to communicate the usefulness of the composition of the invention in the kit for effecting alleviation of the various diseases or disorders recited herein. Optionally, or alternately, the instructional material may describe one or more methods of alleviating the diseases or disorders in a cell or a tissue of a mammal. The instructional material of the kit may, for example, be affixed to a container which contains the identified compound or be shipped together with a container which contains the identified compound. Alternatively, the instructional material may be shipped separately from the container with the intention that the instructional material and the compound be used cooperatively by the recipient.

As used herein, the term "neuroprotective agent" refers to an agent that prevents or slows the progression of neuronal degeneration and/or prevents neuronal cell death.

As used herein, the term "substantially free of", refers to an enantiomeric excess greater than 80%, preferably greater than 90%, more preferably greater than 95%, and most preferably greater than 98%.

As used herein, the term "halogen" or "halo" refers to bromo, chloro, fluoro, and iodo.

The term "haloalkyl" as used herein refers to an alkyl radical bearing at least one halogen substituent, for example, chloromethyl, fluoroethyl or trifluoromethyl and the like.

The term "C₁-Cₙ alkyl" wherein n is an integer, as used herein, represents a branched or linear alkyl group having from one to the specified number of carbon atoms. Typically, C₁-C₆ alkyl groups include, but are not limited to, methyl, ethyl, n-propyl, iso-propyl, butyl, iso-butyl, sec-butyl, tert-butyl, pentyl, hexyl, and the like.

The term "C₂-Cₙ alkenyl" wherein n is an integer, as used herein, represents an olefinically unsaturated branched or linear group having from 2 to the specified number of carbon atoms and at least one double bond. Examples of such groups include, but are not limited to, 1-propenyl, 2-propenyl, 1,3-butadienyl, 1-butenyl, hexenyl, pentenyl, and the like.

The term "C₃-Cₙ cycloalkyl" refers to cyclic alkanes. Exemplary cyclic alkanes include, but are not limited to, cyclopropyl, cyclobutyl, cyclopentyl, cyclohexyl, cycloheptyl, and cyclooctyl.

As used herein, the term "optionally substituted" refers to from zero to four substituents, wherein the substituents are each independently selected. Each of the independently selected substituents may be the same or different than other substituents.

As used herein, the term "stereoisomers" refers to compounds made up of the same atoms bonded by the same bonds but having different spatial structures which are not interchangeable. The three-dimensional structures are called configurations. As used herein, the term "enantiomers" refers to two stereoisomers whose molecules are nonsuperimposable mirror images of one another. As used herein, the term "optical isomer" is equivalent to the term "enantiomer". The terms "racemate", "racemic mixture" or "racemic modification" refer to a mixture of equal parts of enantiomers. The term "chiral center" refers to a carbon atom to which four different groups are attached, as distinguished from prochiral centers. The term "enantiomeric enrichment" as used herein refers to the increase in the amount of one enantiomer as compared to the other. Enantiomeric enrichment is readily determined by one of ordinary skill in the art using standard techniques and procedures, such as gas or high performance liquid chromatography with a chiral column. Choice of the appropriate chiral column, eluent and conditions necessary to effect separation of the enantiomeric pair is well within the knowledge of one of ordinary skill in the art using standard techniques well known in the art, such as those described by J. Jacques, et al., "Enantiomers, Racemates, and Resolutions", John Wiley and Sons, Inc., 1981. Examples of resolutions include recrystallization of diastereomeric salts/derivatives and/or preparative chiral chromatography.

### II. Compositions

### A. Tetrahydrobenzathiazoles

Tetrahydrobenzathiazole has the chemical formula shown below. 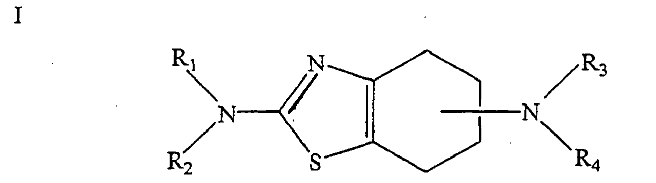 wherein R₁, R₂, R₃, and R₄, are independently selected from the group consisting of H, C₁-C₃ alkyl and C₁-C₃ alkenyl and, the NR₃R₄ group is in the 6-position. In another embodiment R₁, R₂ and R₄ are H, R₃ is C₁-C₃ alkyl, and the NR₃R₄ group is in the 6-position. In another embodiment the compound has the general structure of formula I, wherein R₁ and R₂ are each H and R₃ and R₄ are H or C₁-C₃ alkyl. Pramipexole is where R₁ and R₂ are each H and one of R₃ and R₄ is H and the other is n-propyl.

Pramipexole (PPX, 2-amino-4,5,6,7-tetrahydro-6-propylaminobenzathiazole) exists as two stereoisomers:

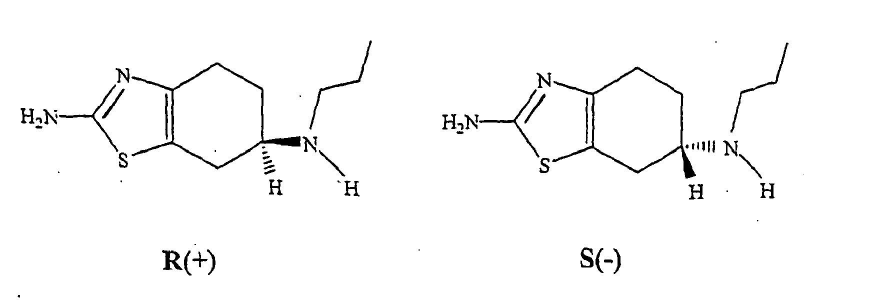

R(+) pramipexole is water soluble and is stable in aqueous solutions.

In one embodiment, the composition contains the R(+) enantiomer of pramipexole, alone or in combination with the S(-) enantiomer of pramipexole, or pharmaceutically acceptable salts thereof. In another embodiment, the enantiomeric excess of the R(+) enantiomer is greater than 80%, preferably greater than 90%, more preferably greater than 95%, and most preferably greater than 99% .

The S(-) enantiomer is a potent agonist of the D2 family of dopamine receptors and is extensively used in the symptomatic management of PD. The synthesis, formulation, and administration of pramipexole are described in U.S. Patent Nos. 4,843,086 and 4,886.812 to Griss et al. and 5,112,842 to Zierenberg et al. S(-) PPX has been shown by several groups to be neuroprotective in cellular and animal models of increased oxidative stress, including MPTP toxicity to dopamine neurons (see U.S. Patent Nos. 5,650,420 and 6,156,777 to Hall et al.). S(-) PPX also reduces oxidative stress produced by the parkinsonian neurotoxin and ETC complex I inhibitor methylpyridinium (MPP+), both *in vitro* and *in vivo,* and can block the opening of the mitochondrial transition pore (MTP) induced by MPP+ and other stimuli (Cassarino, et al, J. Neurochem., Jul., 71(1), 295-301 (1998)). It is believed that the lipophilic cationic structure of PPX allows PPX to cross more easily into the mitochondria, and that this property, in combination with the low reduction potential (320 mV) of PPX, may account for these desirable neuroprotective properties.

Dosing with S(-) PPX is limited in humans due to its potent dopamine agonist properties and therefore limits achievable drug levels in the brain. Because the R(+) enantiomer of PPX has very little dopamine agonist activity (Schneider and Mierau, J. Med. Chem. 30:494-498 (1987)), but may retain the desirable molecular/antioxidant properties of S(-) PPX, this compound may have utility as an effective inhibitor of the activation of cell death cascades and loss of viability that occurs in neurodegenerative diseases.

The compounds may be administered as the free base. However, the compounds are typically administered as a pharmaceutically acceptable acid-addition salt. As used herein, "pharmaceutically acceptable salts" refer to derivatives of the compounds wherein the parent compound is modified by making the acid addition salt thereof. Examples of pharmaceutically acceptable acid-addition salts include, but are not limited to, mineral or organic acid salts of basic residues such as amines. The pharmaceutically acceptable salts include the conventional non-toxic salts or the quaternary ammonium salts of the parent compound formed, for example, from non-toxic inorganic or organic acids. Such conventional non-toxic salts include, but are not limited to, those derived from inorganic acids such as hydrochloric, hydrobromic, sulfuric, sulfamic, phosphoric, and nitric acids; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, tolunesulfonic, naphthalenesulfonic, methanesulfonic, ethane disulfonic, oxalic, and isethionic salts.

The pharmaceutically acceptable salts of the compounds can be synthesized from the parent compound, which contains a basic moiety, by conventional chemical methods. Generally, such salts can be prepared by reacting the free base forms of these compounds with a stoichiometric amount of the appropriate acid in water or in an organic solvent, or in a mixture of the two; generally, non-aqueous media like ether, ethyl acetate, ethanol, isopropanol, or acetonitrile are preferred. Lists of suitable salts are found in Remington's Pharmaceutical Sciences, 20th ed., Lippincott Williams & Wilkins, Baltimore, MD, 2000, p. 704; and "Handbook of Pharmaceutical Salts: Properties, Selection, and Use," P. Heinrich Stahl and Camille G. Wermuth, Eds., Wiley-VCH, Weinheim, 2002.

As generally used herein "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

### B. Combinations with Other Active Agents

The tetrahydrobenzathiazole may be administered adjunctively with other active compounds. Examples include analgesics, anti-inflammatory drugs, antihistamines, antioxidants, vitamins, antivirals, antibiotics, anti-angiogenic agents, antiemetics, scavengers such as cyanide and cyanate scavengers, and other drugs that may be beneficial for the treatment of neurodegenerative disorders.

By adjunctive administration is meant simultaneous administration of the compounds, in the same dosage form, simultaneous administration in separate dosage forms, and separate administration of the compounds.

### C. Carriers, Additives, and Excipients

Formulations are prepared using a pharmaceutically acceptable "carrier" composed of materials that are considered safe and effective and may be administered to an individual without causing undesirable biological side effects or unwanted interactions. The "carrier" is all components present in the pharmaceutical formulation other than the active ingredient or ingredients. The term "carrier" includes, but is not limited, to diluents, binders, lubricants, disintegrators, fillers, and coating compositions.

"Carrier", as used herein, also includes all components of the coating composition which may include plasticizers, pigments, colorants, stabilizing agents, and glidants.

Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name Eudragit® (Roth Pharma, Westerstadt, Germany), Zein, shellac, and polysaccharides. Additionally, the coating material may contain conventional carriers such as plasticizers, pigments, colorants, glidants, stabilization agents, pore formers and surfactants.

Optional pharmaceutically acceptable excipients present in the composition include, but are not limited to, diluents, binders, lubricants, disintegrants, colorants, stabilizers, and surfactants.

Diluents, also termed "fillers," are typically necessary to increase the bulk of a solid dosage form so that a practical size is provided for compression of tablets or formation of beads and granules. Suitable diluents include, but are not limited to, dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powder sugar.

Binders are used to impart cohesive qualities to a solid dosage formulation, and thus ensure that a tablet or bead or granule remains intact after the formation of the dosage forms. Suitable binder materials include, but are not limited to, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydorxypropylmethylcellulose, hydroxypropylcellulose, ethylcellulose, and veegum, and synthetic polymers such as acrylic acid and methacrylic acid copolymers, methyl methacrylate copolymers, aminoalkyl methacrylate copolymers, polyacrylic acid/polymethacrylic acid and polyvinylpyrrolidone.

Lubricants are used to facilitate tablet manufacture. Examples of suitable lubricants include, but are not limited to, magnesium stearate, calcium stearate, stearic acid, glycerol behenate, polyethylene glycol, talc, and mineral oil.

Disintegrants are used to facilitate dosage form disintegration or "breakup" after administration, and generally include, but are not limited to, starch, sodium starch glycolate, sodium carboxymethyl starch, sodium carboxymethylcellulose, hydroxypropyl cellulose, pregelatinized starch, clays, cellulose, alginine, gums or cross linked polymers, such as crosslinked polyvinylpyrrolidone (PVP) (Polyplasdone XL from GAF Chemical Corp).

Stabilizers are used to inhibit or retard drug decomposition reactions which include, by way of example, oxidative reactions.

Surfactants may be anionic, cationic, amphoteric or nonionic surface active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene, and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer 401, stearoyl monoisopropanolamide, and polyoxyethylene hydrogenated tallow amide. Examples of amphoteric surfactants include sodium N-dodecyl-alanine, sodium N-lauryl-beta.-iminodipropionate, myristoamphoacetate, lauryl betaine and lauryl sulfobetaine.

If desired, the compositions may also contain minor amounts of nontoxic auxiliary substances such as wetting or emulsifying agents, dyes, pH buffering agents, and preservatives.

In one embodiment, the tetrahydrobenzathiazole is formulated with a pharmaceutically acceptable carrier and 0.1-1/0% albumin. Albumin functions as a buffer and improves the solubility of the tetrahydrobenzathiazole.

### D. Modified Release Formulations

The compositions described herein can be formulation for modified or sustained release. Examples of modified release compositions include extended release compositions, delayed release compositions, and pulsatile release compositions.

### i. Extended Release Formulations

Extended release formulations are generally prepared as diffusion or osmotic systems, for example, as described in "Remington--The Science and Practice of Pharmacy" (20th ed., Lippincott Williams & Wilkins, Baltimore, Md., 2000). A diffusion system typically consists of two types of devices, reservoir devices and matrix devices, both of which are well known and described in the art. The matrix devices are generally prepared by compressing the drug with a slowly dissolving polymer carrier into a tablet form. The three major types of materials used in the preparation of matrix devices are insoluble plastics, hydrophilic polymers, and fatty compounds. Plastic matrices include, but are not limited to, methyl acrylate-co-methyl methacrylate, polyvinyl chloride, and polyethylene. Hydrophilic polymers include, but are not limited to, methylcellulose, hydroxypropylcellulose, hydroxypropylmethylcellulose, sodium carboxymethylcellulose, and CARBOPOL® 934, and polyethylene oxides. Fatty compounds include, but are not limited to, various waxes such as carnauba wax and glyceryl tristearate.

Alternatively, extended release formulations can be prepared using osmotic systems or by applying a semi-permeable coating to the dosage form. In the latter case, the desired drug release profile can be achieved by combining low permeable and high permeable coating materials in suitable proportion.

The devices with different drug release mechanisms described above can be combined in a final dosage form comprising single or multiple units. Examples of multiple units include multilayer tablets and capsules containing tablets, beads, granules, etc.

An immediate release portion can be added to the extended release system by means of either applying an immediate release layer on top of the extended release core using coating or compression processes or in a multiple unit system, such as a capsule, containing extended and immediate release beads.

Extended release tablets containing hydrophilic polymers are prepared by techniques commonly known in the art such as direct compression, wet granulation, or dry granulation processes. Their formulations usually incorporate polymers, diluents, binders, and lubricants as well as the active pharmaceutical ingredient. The usual diluents include inert powdered substances such as any of many different kinds of starch, powdered cellulose, especially crystalline and microcrystalline cellulose, sugars such as fructose, mannitol and sucrose, grain flours and similar edible powders. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders include substances such as starch, gelatin and sugars such as lactose, fructose, and glucose. Natural and synthetic gums, including acacia, alginates, methylcellulose, and polyvinylpyrrolidine can also be used. Polyethylene glycol, hydrophilic polymers, ethylcellulose and waxes can also serve as binders. A lubricant is necessary in a tablet formulation to prevent the tablet and punches from sticking in the die. The lubricant is chosen from such slippery solids as talc, magnesium and calcium stearate, stearic acid and hydrogenated vegetable oils.

Extended release tablets containing wax materials are generally prepared using methods known in the art such as a direct blend method, a congealing method, and an aqueous dispersion method. In a congealing method, the drug is mixed with a wax material and either spray-congealed or congealed, screened, and processed.

### ii. Delayed Release Dosage Forms

Delayed release formulations are created by coating a solid dosage form with a film of a polymer which is insoluble in the acid environment of the stomach, and soluble in the neutral environment of small intestines.

The delayed release dosage units can be prepared, for example, by coating a drug or a drug-containing composition with a selected coating material. The drug-containing composition may be, e.g., a tablet for incorporation into a capsule, a tablet for use as an inner core in a "coated core" dosage form, or a plurality of drug-containing beads, particles or granules, for incorporation into either a tablet or capsule. Preferred coating materials include bioerodible, gradually hydrolyzable, gradually watersoluble, and/or enzymatically degradable polymers, and may be conventional "enteric" polymers. Enteric polymers, as will be appreciated by those skilled in the art, become soluble in the higher pH environment of the lower gastrointestinal tract or slowly erode as the dosage form passes through the gastrointestinal tract, while enzymatically degradable polymers are degraded by bacterial enzymes present in the lower gastrointestinal tract, particularly in the colon. Suitable coating materials for effecting delayed release include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the tradename Eudragit® (Rohm Pharma; Westerstadt, Germany), including Eudragit® L30D-55 and L100-55 (soluble at pH S.5 and above), Eudragit® L-100 (soluble at pH 6.0 and above), Eudragit® S (soluble at pH 7.0 and above, as a result of a higher degree of esterification), and Eudragits® NE, RL and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylose and guar gum; zein and shellac. Combinations of different coating materials may also be used. Multi-layer coatings using different polymers may also be applied.

The preferred coating weights for particular coating materials may be readily determined by those skilled in the art by evaluating individual release profiles for tablets, beads and granules prepared with different quantities of various coating materials. It is the combination of materials, method and form of application that produce the desired release characteristics, which one can determine only from the clinical studies.

The coating composition may include conventional additives, such as plasticizers, pigments, colorants, stabilizing agents, glidants, etc. A plasticizer is normally present to reduce the fragility of the coating, and will generally represent about 10 wt. % to 50 wt. % relative to the dry weight of the polymer. Examples of typical plasticizers include polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil and acetylated monoglycerides. A stabilizing agent is preferably used to stabilize particles in the dispersion. Typical stabilizing agents are nonionic emulsifiers such as sorbitan esters, polysorbates and polyvinylpyrrolidone. Glidants are recommended to reduce sticking effects during film formation and drying, and will generally represent approximately 25 wt. % to 100 wt. % of the polymer weight in the coating solution. One effective glidant is talc. Other glidants such as magnesium stearate and glycerol monostearates may also be used. Pigments such as titanium dioxide may also be used. Small quantities of an anti-foaming agent, such as a silicone (e.g., simethicone), may also be added to the coating composition.

### III. Methods of Administration

Formulations can be used for restoring neuronal, muscular (cardiac and striated) and/or retinal tissue function in children and adults afflicted with chronic neurodegenerative diseases, such as neurodegenerative movement disorders and ataxias, seizure disorders, motor neuron diseases, and inflammatory demyelinating disorders. Examples of disorders include Alzheimer's disease (AD), Parkinson's disease (PD), and amyotrophic lateral sclerosis (ALS).

A number of central nervous system diseases and conditions result in neuronal damage and each of these conditions can be treated with an effective amount of the tetrahydrobenzathiazole compositions to provide restoration of some function. Conditions which can lead to nerve damage include: Primary neurodegenerative disease; Huntington's Chorea; Stroke and other hypoxic or ischemic processes; neurotrauma; metabolically induced neurological damage; sequelae from cerebral seizures; hemorrhagic stroke; secondary neurodegenerative disease (metabolic or toxic); Parkinson's disease, Alzheimer's disease, Senile Dementia of Alzheimer's Type (SDAT); age associated cognitive dysfunctions; or vascular dementia, multi-infarct dementia, Lewy body dementia, or neurodegenerative dementia.

U.S. Patent Application Publication No. 2005/0032856 to Bennett describes compositions containing pramipexole and the use of such compositions to treat neurodegenerative diseases such as amyotrophic lateral sclerosis (ALS). The compositions can be used to prevent and/or delay symptoms, or alleviate symptoms, related to a variety of neurodegenerative diseases. The compositions contain one of the two stereoisomers of pramipexole. In one embodiment, the compositions contain the R(+) enantiomer, or a pharmaceutically acceptably salt thereof, substantially free of the S(-) enantiomer. The neuroprotective effect of the compounds derives at least in part from the active compound's ability to prevent neural cell death by at least one of three mechanisms. First, the tetrahydrobenzathiazoles are capable of reducing the formation of a reactive oxygen species (ROS) (both *in vivo* in rat brain and *in vitro* in cells with impaired mitochondrial energy production) induced with neurotoxins that can mimic Parkinson's disease. In this manner the tetrahydrobenzathiazoles function as "free radical scavengers." Decreased serum ROS levels can be detected by measuring the conversion of salicylate to 2,3-dihydroxybenzoic acid (2,3 DHB) (Floyd et al., J. Biochem. Biophys. Methods, 10:221-235 (1984); Hall et al., J. Neurochem., Vol. 60, 588-594 (1993)). Second, the tetrahydrobenzathiazoles can partially restore the reduced ΔΨ that is correlated with Alzheimer's disease and Parkinson's disease mitochondria. Third, the tetrahydrobenzathiazoles can block the apoptotic cell death pathways which are produced by pharmacological models of Alzheimer's disease and Parkinson' disease mitochondrial impairment.

The dosage to be used is dependent on the specific disorder to be treated, as well as additional factors including the age, weight, general state of health, severity of the symptoms, frequency of the treatment and whether additional active agent are co-administered with the tetrahydrobenzathiazole. The amounts of the individual active compounds are easily determined by routine procedures known to those of ordinary skill in the art. For example, the tetrahydrobenzathiazoles described herein can be administered orally to humans with NDD at a dose of 0.1-300 mg/kg/daily, preferably 0.5-50 mg/kg/daily, and most preferably 1-10 mg/kg/daily or 30 mg/daily. Daily total doses administered orally are typically between 10 mg and 500 mg. Alternatively, the tetrahydrobenzathiazoles can be administered parenterally to humans with acute brain injury in single doses between 10 mg and 100 mg, and/or by continuous intravenous infusions between 10 mg/day and 500 mg/day.

The compositions can be administered to an individual in need thereof by any number of routes including, but not limited to, topical, oral, intravenous, intramuscular, intra-arterial, intramedullary, intrathecal, intraventricular, transdermal, subcutaneous, intraperitoneal, intranasal, enteral, topical, sublingual, or rectal means. The composition can be administered orally on a chronic basis for preventing neural cell loss in NDD (and more particularly reducing oxidative stress in ALS patients), or it can be formulated and administered intravenously for prevention of neural cell loss in acute brain injury, such as that resulting from strokes, sub-arachnoid hemorrhage, hypoxic-ischemic brain injury, status eplepticus, traumatic brain injury, and hypoglycemic brain injury).

A kit is provided containing a pharmaceutical composition containing the R(+) enantiomer of pramipexole. The compositions can be formulated for immediate and/or modified release. The packaging material may be a box, bottle, blister package, tray, or card. The kit will include a package insert instructing the patient to take a specific dose at a specific time, for example, a first dose on day one, a second higher dose on day two, a third higher dose on day three, and so on, until a maintenance dose is reached. The compounds can be packaged in a single dosage unit or a multidosage unit.

### Examples

### Example 1. Clinical Studies of R(+) Pramipexole

Phase I clinical studies with R(+) pramipexole were conducted in patients with amyotrophic lateral sclerosis (ALS), a fatal adult neurodegenerative disease arising from the loss of motor nerve cells. During the studies, it was found that the approximately half the patients treated with 30 mg/day of R(+) pramipexole for up to 8 weeks reported improvements in motor function. These reports included such things as regaining the ability to speak coherently, the ability to climb stairs again, and improved motor skills in their extremities, i.e. hands and/or fingers. This data indicates that rather than simply slowing the loss of function, R(+) pramipexole improves and restores function in an otherwise rapidly progressive neurodegenerative disease.

## Claims

1. A pharmaceutical composition comprising a tetrahydrobenzathiazole having the chemical formula shown below, or its pharmaceutically acceptable salt, for use in a method of restoring neuronal, muscular (cardiac and striated) and/or retinal tissue function in children or adults with neurodegenerative diseases: 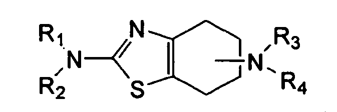 wherein R₁, R₂, R₃ and R₄ are independently selected from the group consisting of H, C₁-C₃ alkyl and C₁-C₃ alkenyl and the -N(R₃)(R₄) group is in the 6-position.

2. The pharmaceutical composition of claim 1, wherein the enantiomeric excess of the R(+) enantiomer is greater than 99%.

3. The pharmaceutical composition of claims 1 or 2, wherein the tetrahydrobenzathiazole is the R(+) enantiomer of pramipexole.

4. The pharmaceutical composition of any one of claims 1 to 3, wherein the composition is suitable for topical, transdermal, enteral, or parenteral administration.

5. The pharmaceutical composition according to claim 4, wherein the tetrahydrobenzathiazole or its pharmaceutically acceptable salt is provided in a dosage formulation for enteral administration of a single dosage between 10 mg and 100 mg.

6. The pharmaceutical composition of claim 4, wherein the tetrahydrobenzathiazole or its pharmaceutically acceptable salt is provided in a dosage formulation for parenteral administration of a single dosage by continuous intravenous infusions of between 10 mg/day and 500 mg/day.

7. The pharmaceutical composition of claim 5. wherein the dosage formulation comprises 30 mg of pramipexole.

8. The pharmaceutical composition of any of the preceding claims, wherein the neurodegenerative disease is Alzheimer's disease, Parkinson's disease, or amyotrophic lateral sclerosis.

## Patentansprüche

1. Pharmazeutische Zusammensetzung, umfassend ein Tetrahydrobenzathiazol mit der nachstehend gezeigten chemischen Formel oder sein pharmazeutisch akzeptables Salz, zur Verwendung in einem Verfahren zur Wiederherstellung der Funktion von neuronalem, muskulärem (cardialem und gestreiftem) und/oder retinalem Gewebe bei Kindern oder Erwachsenen mit neurodegenerativen Krankheiten: 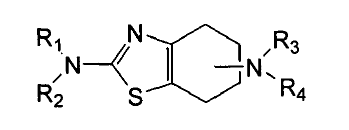 worin R₁, R₂, R₃ und R₄ unabhängig ausgewählt sind aus der Gruppe bestehend aus H, C₁-C₃-Alkyl und C₁-C₃-Alkenyl und die -N(R₃)(R₄)-Gruppe an der 6-Position vorliegt.

2. Pharmazeutische Zusammensetzung gemäß Anspruch 1, worin der Enantiomerenüberschuss des R(+)-Enantiomers größer als 99% ist.

3. Pharmazeutische Zusammensetzung gemäß Anspruch 1 oder 2, worin das Tetrahydrobenzathiazol das R(+)-Enantiomer von Pramipexol ist.

4. Pharmazeutische Zusammensetzung gemäß irgendeinem der Ansprüche 1 bis 3, worin die Zusammensetzung für die topische, transdermale, enterale oder parenterale Verabreichung geeignet ist.

5. Pharmazeutische Zusammensetzung gemäß Anspruch 4, worin das Tetrahydrobenzathiazol oder sein pharmazeutisch akzeptables Salz in einer Dosierungsformulierung für die enterale Verabreichung einer Einzeldosis zwischen 10 mg und 100 mg bereitgestellt ist.

6. Pharmazeutische Zusammensetzung gemäß Anspruch 4, worin das Tetrahydrobenzathiazol oder sein pharmazeutisch akzeptables Salz in einer Dosierungsformulierung für die parenterale Verabreichung einer Einzeldosis durch kontinuierliche intravenöse Infusionen zwischen 10 mg/Tag und 500 mg/Tag bereitgestellt ist.

7. Pharmazeutische Zusammensetzung gemäß Anspruch 5, worin die Dosierungsformulierung 30 mg Pramipexol umfasst.

8. Pharmazeutische Zusammensetzug gemäß irgendeinem der vorhergehenden Ansprüche, worin die neurodegenerative Krankheit die Alzheimer-Krankheit, die Parkinson-Krankheit oder amyotrophe Lateralsklerose ist.

## Revendications

1. Composition pharmaceutique comprenant un tétrahydrobenzathiazole ayant la formule chimique présentée ci-dessous, ou son sel pharmaceutiquement acceptable, pour une utilisation dans un procédé de restauration de la fonction d'un tissu neuronal, musculaire (cardiaque et strié) et/ou rétinien chez des enfants ou des adultes avec des maladies neurodégénératives : 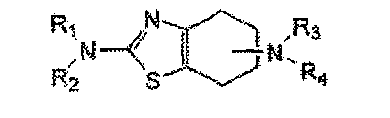 dans laquelle R₁, R₂, R₃ et R₄ sont choisis, de manière indépendante, dans le groupe constitué de H, un alkyle en C₁-C₃ et un alcényle en C₁-C₃ et le groupe -N(R₃) (R₄) est dans la position 6.

2. Composition pharmaceutique selon la revendication 1, dans laquelle l'excès énantiomérique de l'énantiomère R(+) est supérieur à 99 %.

3. Composition pharmaceutique selon la revendication 1 ou 2, dans laquelle le tétrahydrobenzathiazole est l'énantiomère R(+) du pramipexole.

4. Composition pharmaceutique selon l'une quelconque des revendications 1 à 3, dans laquelle la composition est convenable pour une administration topique, transdermique, entérale, ou parentérale.

5. Composition pharmaceutique selon la revendication 4, dans laquelle le tétrahydrobenzathiazole ou son sel pharmaceutiquement acceptable est fourni dans une formulation posologique pour l'administration entérale d'une dose unique entre 10 mg et 100 mg.

6. Composition pharmaceutique selon la revendication 4, dans laquelle le tétrahydrobenzathiazole ou son sel pharmaceutiquement acceptable est fourni dans une formulation posologique pour l'administration parentérale d'une dose unique par des perfusions intraveineuses continues entre 10 mg/jour et 500 mg/jour.

7. Composition pharmaceutique selon la revendication 5, dans laquelle la formulation posologique comprend 30 mg de pramipexole.

8. Composition pharmaceutique selon l'une quelconque des revendications précédentes, dans laquelle la maladie neurodégénérative est la maladie d'Alzheimer, la maladie de Parkinson, ou la sclérose latérale amyotrophique.
